# FASCICULE DE BREVET EUROPEEN

(11) **EP 1 423 145 B1**
(45) Date de publication et mention de la délivrance du brevet: **07.06.2006**
(21) Numéro de dépôt: 02772514.2
(22) Date de dépôt: 28.08.2002
(51) Int. Cl.: A61K 45/06, A61P 25/16, A61K 31/4353, A61K 31/397

(54) **COMPOSITIONS POUR LE TRAITEMENT DE LA MALADIE DE PARKINSON CONTENANT UN ANTAGONISTE DU RECEPTEUR CB1 ET UN PRODUIT QUI ACTIVE LA NEUROTRANSMISSION DOPAMINERGIQUE DANS LE CERVEAU**
ZUSAMMENSETZUNGEN ZUR BEHANDLUNG VON PARKINSON'S KRANKHEIT, WELCHE ANTAGONISTEN DER CB1-REZEPTOREN UND AKTIVIERUNGSSUBSTANZEN DER DOPAMIN-NEUROTRANSMISSION IM GEHIRN ENTHALTEN
COMPOSITIONS FOR THE TREATMENT OF PARKINSON'S DISEASE CONTAINING A CB1 RECEPTOR ANTAGONIST AND A PRODUCT THAT ACTIVATES DOPAMINERGIC NEUROTRANSMISSION IN THE BRAIN

(30) Priorité: 29.08.2001 FR 0111200
(43) Date de publication de la demande: 02.06.2004
(60) Demande divisionnaire: 06000097.3
(73) Titulaire: Aventis Pharma S.A., 92160 Antony (FR)
(72) Inventeur: BENAVIDES, Jésus, F-92280 Chatenay-Malabry (FR); BOCCIO, Daniel, F-77310 Pringy (FR); HENIN, Yvette, F-75019 Paris (FR); PIOT-GROSJEAN, Odile, F-94600 Choisy le Roi (FR)
(74) Mandataire: Rousseau, Pierrick Edouard
(86) Numéro de dépôt international: PCT/FR2002/002946
(87) Numéro de publication internationale: WO 2003/020314

(56) Documents cités:
- SANUDO-PENA, M. CLARA ET AL: "A novel neurotransmitter system involved in the control of motor behavior by the basal ganglia" ANN. N. Y. ACAD. SCI. (1998), 860(NEURONAL MECHANISMS FOR GENERATING LOCOMOTOR ACTIVITY), 475-479, XP008002762
- MESCHLER, JUSTIN P. ET AL: "D2, but not D1 dopamine receptor agonists potentiate cannabinoid-induced sedation in nonhuman primates" JOURNAL OF PHARMACOLOGY AND EXPERIMENTAL THERAPEUTICS (2000), 292(3), 952-959, XP008002761
- DI MARZO V ET AL: "Enhanced levels of endogenous cannabinoids in the globus pallidus are associated with a reduction in movement in an animal model of Parkinson's disease." FASEB JOURNAL, (2000 JUL) 14 (10) 1432-8., XP001075032
- BROTCHIE, J. M. ET AL: "The cannabinoid receptor antagonist SR141716A reduces L - DOPA -induced dyskinesia in the MPTP-treated primate model of Parkinson's disease." BRITISH JOURNAL OF PHARMACOLOGY, (MARCH, 1998) VOL. 123, NO. PROC. SUPPL. PP. 66P. MEETING INFO.: MEETING OF THE BRITISH PHARMACOLOGICAL SOCIETY HELD JOINTL WITH DUTCH PHARMACOLOGICAL SOCIETY, THE BELGIAN SOCIETY FOR FUNDAMENTAL AND CLINICAL PHYSIOLO, XP008002758

## Description

La présence invention concerne l'association de un ou plusieurs antagonistes du récepteur CB1 choisi parmi N-{1-[bis-(4-chlorophényl)méthyl]azétidin-3-yl}-N-pyrid-3-yl-méthylsulfonamide, N-{1-[bis-(4-chlorophényl)méthyl]azétidin-3-yl}-N-(3,5-difluorophényl)-méthylsulfonamide, et de un ou plusieurs produits qui activent la neurotransmission dopaminergique dans le cerveau, les compositions pharmaceutiques les contenant et leur utilisation pour le traitement de la maladie de Parkinson.

Des antagonistes du récepteur CB1 sont développés pour le traitement de la schizophrénie (D. KENDALL, Curr. Opin. Cenf. Peripher. Nerv. Syst. Invest. Drugs, 2(1), 112-122, 2000), pour leur action sur la prise alimentaire (G. COLOMBO et coll., Life Sciences, 63 (8), 113-117 (1998); J. SIAMAND et coll., Behavioural Pharmacol., 9, 179-181 (1998)), pour le traitement de la maladie de Parkinson, épilepsie, migraine, stress (G. GERDEMAN, DM. LOVINGER, J.Neurophysiol, 85(1), 468-471, 2001 ; WO0046209).

La maladie de Parkinson résulte d'un désordre neurologique chronique et progressif. Elle repose sur un déficit en dopamine, un excès relatif en acétylcholine et est associée à une destruction des neurones dopaminergiques qui participent au contrôle des activités motrices (H. LULLMANN et coll., Atlas de poche de pharmacologie, 2° Ed, Médecine-Sciences, Flammarion, ISBN2-257-12119-8). Le traitement de la maladie de Parkinson est principalement pharmacologique et fait appel à différents médicaments destinés à accroître la quantité de dopamine présente dans le cerveau.

La dopamine ne traversant pas la barrière hémato-encéphalique, la lévodopa, précurseur de la dopamine convertie en dopamine par la dopa-décarboxylase, a été développée dans les années 60. La lévodopa reste aujourd'hui le premier traitement de choix de la maladie de Parkinson et donne initialement de bons résultats mais après plusieurs années, on observe chez la majorité des patients des fluctuations de réponse (effet 'on-off'), une diminution de son efficacité au fur et à mesure que la maladie progresse (effet 'wearing-off', détérioration de fin de dose), et surtout des dyskinésies (mouvements anormaux involontaires). Un état de psychose peut également être observé.

D'autres médicaments comme les agonistes dopaminergiques sont également préconisés seuls ou en association à la lévodopa et ont principalement pour objet de réduire au minimum les effets indésirables de celle-ci. Depuis quelques années, des inhibiteurs sélectifs de la monoamine oxydase MAO-B, enzyme de dégradation de la dopamine dans le cerveau, ainsi que des inhibiteurs de la catéchol-O méthyl-transférase (COMT), enzyme qui empêche la lévodopa de franchir la barrière hémato-encéphalique, ont été développés et prescrits en association avec la lévodopa. Des effets secondaires importants ont également été observés avec ces thérapies.

Afin de remédier aux inconvénients susmentionnés, il a été trouvé que l'association de un ou plusieurs antagonistes du récepteur CB1 et de un ou plusieurs produits qui activent la neurotransmission dopaminergique dans le cerveau présente un effet de synergie dans le traitement de la maladie de Parkinson. En effet cette association permettrait de potentialiser les effets symptomatiques d'une monothérapie dopaminergique (lévodopa, agonistes dopaminergiques et inhibiteurs d'enzyme) et permettrait de réduire les effets secondaires, en particulier les dyskinésies.

Outre la lévodopa, précurseur de la dopamine, on peut citer parmi les agonistes dopaminergiques, les produits suivants : bromocriptine (Novartis), cabergoline (Pharmacia Corp.) adrogolide (Abbott Laboratories), BAM-1110(Maruko Seiyaku Co Ltd), Duodopa® (Neopharma), L-dopa, dopadose (Neopharma), CHF1512 (Chiesi), NeuroCell-PD (Diacrin Inc), PNU-95666 (Pharmacia & Upjohn), ropinirole (GlaxoSmithKline Beecham), pramipexole (Boehringer Ingelheim) rotigotine (Discovery Therapeutics, Lohmann Therapie System), spheramine (Titan Pharmaceuticals), TV 1203 (Teva pharmaceutical), uridine (Polifarma).

Parmi les inhibiteurs de MAO_{B}, on peut citer: rasagiline (Teva Pharmaceutical Ind.) selegiline (RPScherer Corp / Elan) SL340026 (Sanofi-Synthelabo).

Parmi les inhibiteurs de COMT, on peut citer: tolcapone (Roche) et entacapone (Orion Pharma).

L'invention a donc pour objet l'association de un ou plusieurs produits activant la neurotransmission dopaminergique dans le cerveau et de un ou plusieurs dérivés d'azétidine antagonistes CB1 choisi parmi N-{1-[bis-(4-chlorophényl)méthyl]azétidin-3-yl}-N-pyrid-3-yl-méthylsulfonamide, N-{1-[bis-(4-chlorophényl)méthyl]azétidin-3-yl}-N-(3,5-difluorophényl)-méthylsulfonamide, leurs isomères optiques et leurs sels pharmaceutiquement acceptables.

Comme exemples de sels pharmaceutiquement acceptables des dérivés d'azétidine, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-oxynaphtoate, nitrate, oxalate, pamoate, phosphate, salicylate, succinate, sulfate, tartrate, théophyllinacétate et p-toluènesulfonate.

Les énantiomères des composés tels que définis précédemment peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-β-oxynaphtoate, nitrate, oxalate, pamoate, phosphate, salicylate, succinate, sulfate, tartrate, théophyllinacétate et p-toluènesulfonate.

### Exemple1 :

Le N-{1-[bis-(4-chlorophényl)méthyl]azétidin-3-yl}-N-pyrid-3-yl-méthylsulfonamide peut être préparé en opérant de la façon suivante : A une solution de 0,144 g de N-{1-[bis-(4-chlorophényl)méthyl]azétidin-3-yl}-N-(1-oxyde-pyrid-3-yl)-méthylsulfonamide dans 5 cm³ de chloroforme, on coule 0,042 cm³ de trichlorure de phosphore, puis chauffe le mélange à la température du reflux. Après 1 heure et 30 minutes d'agitation, le mélange réactionnel est laissé revenir à température ordinaire, puis est additionné de 5 cm³ d'acide chlorhydrique 0,1N, puis agité et décanté. La phase organique est diluée avec 20 cm³ de chloroforme, séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 9 cm, diamètre 1,8 cm), en éluant sous une pression de 0,1 bar d'argon avec un mélange de dichlorométhane et de méthanol (95/5 en volumes) et en recueillant des fractions de 15 cm³. Les fractions 2 à 4 sont réunies et concentrées à sec sous pression réduite (2,7 kPa). Le résidu est agité avec 15 cm³ d'oxyde de diéthyle, la suspension est filtrée, le solide essoré puis séché sous pression réduite (2,7 kPa). On obtient 35 mg de N-{1-[bis-(4-chlorophényl)méthyl]azétidin-3-yl}-N-pyrid-3-yl-méthylsulfonamide, sous la forme d'un solide crème [Spectre de R.M.N. ¹H (300 MHz, CDCl₃, δ en ppm) : de 2,80 à 2,95 (mt : 2H); 2,87 (s : 3H); 3,51 (t dédoublé, J = 7 et 1,5 Hz : 2H); 4,18 (s : 1H); 4,65 (mt : 1H); de 7,15 à 7,35 (mt : 8H); 7,37 (dd large, J = 8 et 5 Hz : 1H); 7,64 (d démultiplié, J = 8 Hz : 1H); 8 ,52 (d large, J = 2 Hz : 1H); 8,61 (d large, J = 5 Hz : 1H)].

### exemple 2 :

### Méthode 1 :

Le N- {1-[bis-(4-chlorophényl)méthyl]azétidin-3-yl}-N-(3,5-difluorophényl)-méthylsulfonamide peut être préparé en opérant de la façon suivante : A un mélange de 1,23 g de méthylsulfonate de 1-[bis(4-chlorophényl)méthyl]azétidin-3-yle et de 0,66 g de N-(3,5-difluorophényl)méthylsulfonamide, dans 25 cm³ de dioxane, on ajoute 1,0 g de carbonate de césium. Après 5 heures d'agitation à la température du reflux puis 20 heures à 20°C, le mélange réactionnel est additionné de 50 cm³ d'oxyde de diéthyle et de 30 cm³ de saumure, puis est agité et décanté. La phase organique est séchée sur sulfate de magnésium, filtrée puis concentrée à sec à 50°C sous pression réduite (2,7 kPa). L'huile orange obtenue est chromatographiée sur une colonne de gel de silice (granulométrie 0,040-0,063 mm, hauteur 25 cm, diamètre 2,0 cm), en éluant sous une pression de 0,5 bar d'argon avec un mélange de cyclohexane et d'acétate d'éthyle (65/35 en volumes) et en recueillant des fractions de 10 cm³. Les fractions 6 à 10 sont réunies et concentrées à sec sous pression réduite (2,7 Kpa). Le résidu est chromatographié sur une colonne de gel de silice (granulométrie 0,040-0,063 mm, hauteur 15 cm, diamètre 1,0 cm), en éluant sous une pression de 0,5 bar d'argon avec un mélange de cyclohexane et d'acétate d'éthyle (65/35 en volumes) et en recueillant des fractions de 5 cm³. La fraction 7 est concentrée à sec sous pression réduite (2,7 kPa). On obtient 0,11 g de N-{1-[bis-(4-chlorophényl)méthyl]azétidin-3-yl}-N-(3,5-difluorophényl)-méthylsulfonamide, sous la forme d'une poudre blanche [Spectre de R.M.N ¹H (300 MHz, CDCl₃, δ en ppm) : 2,82 (s : 3H); 2,85 (mt : 2H); 3,52 (t dédoublé, J = 7 et 2 Hz : 2H); 4,22 (s : 1H); 4,47 (mt : 1H); de 6,75 à 6,90 (mt :3H); de 7,20 à 7,35 (mt : 8H)].

### Méthode 2

A une solution de 1,41 g de 1-[bis-(4-chlorophényl)méthyl]azétidin-3-ol et de 0,95 g de N-(3,5-difluorophényl)méthylsulfonamide dans 100 cm³ de tétrahydrofuranne anhydre, on ajoute sous argon 0,78 cm³ d'azodicarboxylate de diéthyle et 1,31 g de triphénylphosphine. Après 16 heures d'agitation à 20°C, 300 cm³ d'acétate d'éthyle sont additionnés, le mélange réactionnel est lavé 2 fois avec 100 cm³ d'eau, séché sur sulfate de magnésium et concentré à sec sous pression réduite (2,7 kPa). Le résidu est chromatographié sur une colonne de gel de silice (granulométrie 0,20-0,063 mm, hauteur 50.cm, diamètre 4 cm), en éluant sous une pression de 0,6 bar d'argon avec un mélange de cyclohexane et d'acétate d'éthyle (75/25 en volumes) et en recueillant des fractions de 125 cm³. Les fractions 6 à 12 sont réunies et concentrées à sec sous pression réduite (2,7 kPa). On obtient 1,8 g d'un solide qui est dissous à chaud dans un mélange acétate d'éthyle/diisopropyle éther (15/2 en volume), refroidi, dilué avec 100 cm³ de pentane pour amorcer la cristallisation. Après filtration et séchage, on obtient 1,0g de N-{1-[bis-(4-chlorophényl)méthyl]azétidin-3-yl}-N-(3,5-difluorophényl)-méthylsulfonamide sous la forme de cristaux blancs fondants à 154°C.

Le N-(3,5-difluorophényl)méthylsulfonamide, peut être préparé en opérant de la façon suivante : A une solution de 3,5 g de 3,5-difluoroaniline dans 75 cm³ de dichlorométhane, on ajoute lentement 2,0 cm³ de chlorure de méthylsulfonyle, 3,8 cm³ de triéthylamine et 20 mg de 4-diméthylaminopyridine. Après 20 heures d'agitation à 20°C, le mélange réactionnel, additionné de 20 cm³ de dichlorométhane et de 20 cm³ d'eau, est agité puis décanté. La phase organique est séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 20 cm, diamètre 2,0 cm), en éluant sous une pression de 0,1 bar d'argon avec du dichlorométhane et en recueillant des fractions de 25 cm³. Les fractions 14 à 20 sont réunies et concentrées à sec sous pression réduite (2,7 kPa). On obtient 0,66 g de N-(3,5-difluorophényl)méthylsulfonamide, sous la forme d'une poudre blanche.

Le méthylsulfonate de 1-[bis(4-chlorophényl)méthyl]azétidin-3-yle peut être préparé en opérant de la façon suivante : A une solution de 12 g de 1-[bis-(4-chlorophényl)méthyl]azétidin-3-ol dans 200 cm³ de dichlorométhane, on ajoute sous argon en 10 minutes 3,5 cm³ de chlorure de méthylsulfonyle, puis refroidit à +5°C et coule en 10 minutes 3,8 cm³ de pyridine. Après 30 minutes d'agitation à +5°C puis 20 heures à 20°C, le mélange réactionnel est dilué avec 100 cm³ d'eau et 100 cm³ de dichlorométhane. Le mélange, d'abord filtré est décanté. La phase organique est lavée avec de l'eau, puis séchée sur sulfate de magnésium, filtrée et concentrée à sec sous pression réduite (2,7 kPa). L'huile obtenue est chromatographiée sur une colonne de gel de silice (granulométrie 0,063-0,200 mm, hauteur 40 cm, diamètre 3,0 cm), en éluant sous une pression de 0,5 bar d'argon avec un mélange de cyclohexane et d'acétate d'éthyle (70/30 en volumes) et en recueillant des fractions de 100 cm³. Les fractions 4 à 15 sont réunies et concentrées à sec sous pression réduite (2,7 kPa). On obtient 6,8 g de méthylsulfonate de 1-[bis(4-chlorophényl)méthyl]azétidin-3-yle, sous la forme d'une huile jaune.

Le 1-[bis(4-chlorophényl)méthyl]azétidin-3-ol peut être préparé selon le mode opératoire décrit par KATRITZKY A.R. et coll., J. Heterocycl. Chem., 271 (1994), en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

L'effet de synergie de l'association de un ou plusieurs produits qui activent la neurotransmission dopaminergique dans le cerveau et de un ou plusieurs antagonistes CB1 dans le traitement de la maladie de Parkinson a été déterminé dans un modèle d'akinésie induite par la réserpine chez le rat selon le protocole suivant :

Des rats mâles Sprague-Dawley ont été traités avec la réserpine administrée par voie sous-cutanée à la dose de 3 mg/kg (1ml/kg) afin d'induire une akinésie chez l'animal. 18 heures après ce traitement, l'activité locomotrice de ces animaux a été mesurée et enregistrée à l'aide d'un système automatisé (Videotrack, France). La locomotion, exprimée en centimètres, est estimée par une distance moyenne globale parcourue pendant cette période (n= 11-38 rats par groupe). L'analyse statistique est réalisée par une analyse de variance et une comparaison post-hoc (si approprié) à l'aide d'un test de Mann et Whitney ou de Dunnett. Un effet significatif est noté pour p<0.05.

Les tableaux 1 et 2 démontrent l'effet de synergie de l'association.

Le tableau 1 concerne l'administration ip de l'antagoniste CB1 et le tableau 2 concerne l'administration po de l'antagoniste CB1

Les résultats pour l'administration ip de l'antagoniste CB1 (tableau 1) sont exprimés en pourcentage d'augmentation par rapport à l'activité du quinpirole et en pourcentage de diminution par rapport à l'activité d'une très forte dose de lévodopa

L'association d'un antagoniste du récepteur CB1 et d'un agoniste dopaminergique D2 (quinpirole) est réalisée de la manière suivante:

Le produit antagoniste CB1 (1.5 mg/kg i.p., 2 ml/kg) et le quinpirole (62.5 µg/kg i.p., 1 ml/kg) sont co-administrés 18 heures après l'injection de réserpine. L'enregistrement de l'activité motrice débute 5 minutes après la co-administration des produits et dure 1 heure.

L'association d'un antagoniste du récepteur CB1 et d'une forte dose de lévodopa (modèle de dyskinésie) est réalisée de la manière suivante :

Le produit antagoniste CB1 (3 mg/kg i.p., 2 ml/kg) et la lévodopa (120 mg/kg + bensérazide 50 mg/kg i.p., 5 ml/kg) sont co-administrés. Le bensérazide est un inhibiteur de la dopa-décarboxylase périphérique, ce qui permet à la lévodopa de franchir la barrière hémato-encéphalique avant sa transformation en dopamine. L'enregistrement de l'activité motrice débute 5 minutes après la co-administration et dure 2.5 heures.

**TABLEAU 1**

| Rats réserpinés | Association au quinpirole (62.5 µg/kg ip) | Association à la lévodopa (120 mg/kg ip) |
|---|---|---|
| **Exemple 2** | +139%*** | - 54% *NS* |
| | (1.5 mg/kg i.p.) | (3 mg/kg i.p.) |
| **Exemple 1** | +96% ** | -20% NS (1.5 mg/kg) |
| | | (3 mg/kg non testé) |
| SR141716A | +116%*** | - 61% * |
| 1 mg/kg i.p. | | |

| | | |
|---|---|---|
| SR141716A: N-(piperidin-1-yl)-5(4-chlorophenyl)-1-(2,4-dichlorophenyl)-4-methyl-1H-pyrazole-3-carboxamidehydrochloride | | |
| ANOVA + Mann-Whitney: *p<0.05, **p<0.01, ***p<0.001. | | |

Ces résultats selon l'invention démontrent que les antagonistes du récepteur CB 1:
- potentialisent significativement les effets d'un agoniste dopaminergique D2 (réduction des symptômes parkinsoniens)
- et réduisent l'hyperactivité induite par une très forte dose de lévodopa (activité anti-dyskinétique)

Les études par voie orale sont effectuée dans un solvant hydrophobe de formulation Labrafil/Labrasol (40/60%, w/w). Ces produits sont administrés (sous un volume de 1ml/kg) une heure avant l'agoniste dopaminergique. L'enregistrement de l'activité locomotrice débute 5 min après l'injection intra-péritonéale de l'agoniste dopaminergique et dure 1 heure. L'agoniste dopaminergique D1 est le C1-APB à 0.3 mg/kg. L'agoniste dopaminergique D2 est le quinpirole à 0.1mg.kg.

Les résultats pour l'administration po de l'antagoniste CB1 à trois doses différentes (1, 3 et 10 mg/kg/po) et les résultats (tableau 2) sont exprimés en pourcentage d'augmentation par rapport à l'activité du quinpirole et en pourcentage de diminution par rapport à l'activité d'une forte dose de C1-APB (SKF 82958)

**TABLEAU 2**

| | Dose Mg/kg po | Association au quinpirole (0.1 mg/kg ip) | Association au CI-APB (0.3 mg/kg ip) |
|---|---|---|---|
| **Exemple 2** | 1 | +55% NS | -16% NS |
| | 3 | +62% ***** | -61 % * |
| | 10 | +97% ** | -62% * |
| **Exemple 1** | 1 | -1% NS | +22% NS |
| | 3 | +101%* | -21% NS |
| | 10 | +102% * | -53% * |
| SR141716A | 1 | +57% * NS | -32% NS |
| | 3 | +121% ** | -58% * |
| | 10 | +87% ** | -82%** |

| | | | |
|---|---|---|---|
| SR141716A : N-(piperidin-1-yl)-5(4-chlorophenyl)-1-(2,4-dichlorophenyl)-4-methyl-1H-pyrazole-3-carboxamidehydrochloride | | | |
| ANOVA + Dunnett: *p<0.05, **p<0.01 | | | |

Ces résultats selon l'invention démontrent que les antagonistes du récepteur CB1 :
- potentialisent significativement les effets d'un agoniste dopaminergique D2 (réduction des symptômes parkinsoniens)
- et réduisent l'hyperactivité induite par une forte dose de type D1 (activité anti-dyskinétique)

Les composés de l'association peuvent être employés par voie orale, parentérale, transdermale ou rectale soit simultanément soit séparément soit de façon étalée dans le temps.

La présente invention concerne également les compositions pharmaceutiques contenant l'association de un ou plusieurs produits qui activent la neurotransmission dans le cerveau et de un ou plusieurs antagonistes du récepteur CB1 tels que définis précédemment avec un véhicule pharmaceutiquement acceptable.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pharmaceutiques renfermant l'association telle que défmie précédemment contiennent généralement 0.1 à 500 mg de l'antagoniste CB1. La présente invention concerne également la méthode de traitement de la maladie de Parkinson qui consiste à administrer au patient une association ou une composition pharmaceutique renfermant l'association telle que définie précédemment soit simultanément soit séparément soit de manière étalée dans le temps.

Les doses dépendent de l'effet recherché, de la durée du traitement et de la voie d'administration utilisée; elles sont généralement de 0,1 à 500 mg par jour par voie orale pour un adulte de l'antagoniste CB 1.

D'une façon générale, le médecin déterminera la posologie appropriée en fonction de l'âge, du poids et de tous les autres facteurs propres au sujet à traiter.

## Revendications

1. Association de un ou plusieurs produits qui activent la neurotransmission dopaminergique dans le cerveau et de un ou plusieurs dérivés d'azétidine choisi parmi les composés suivants :
N-{1-[bis-(4-chlorophényl)méthyl]azétidin-3-yl}-N-pyrid-3-yl-méthylsulfonamide,
N-{1-[bis-(4-chlorophényl)méthyl]azétidin-3-yl}-N-(3,5-difluorophényl)-méthylsulfonamide,
leurs sels pharmaceutiquement acceptables

2. Association de un ou plusieurs produits qui activent la neurotransmission dopaminergique dans le cerveau et de un dérivé d'azétidine :
N-{1-[bis-(4-chlorophenyl)méthyl]azétidin-3-yl}-N-pyrid-3-yl-méthylsulfonamide,
leurs sels pharmaceutiquement acceptables

3. Association de un ou plusieurs produits qui activent la neurotransmission dopaminergique dans le cerveau et de un dérivé d'azétidine :
N-{1-[bis-(4-chlorophényl)méthyl]azétidin-3-yl}-N(3,5-difluorophényl)-méthylsulfonamide,
leurs sels pharmaceutiquement acceptables

4. Association selon l'une des revendications 1 à 3 **caractérisée en ce que** le produit qui active la neurotransmission dopaminergique dans le cerveau est choisi parmi les composés suivants :
bromocriptine, cabergoline, Adrogolide, BAM-1110, duodopa, lévodopa, dopadose, CHF1512, PNU-95666, ropinirole, pramipexole, rotigotine, spheramine, TV1203, uridine, rasagiline, selegiline, SL340026, tolcapone, entacapone

5. Association selon les revendications 1 et 2 **caractérisée en ce que** le produit qui active la neurotransmission dopaminergique dans le cerveau est la lévodopa et l'antagoniste CB1 est le N-{1-[bis-(4-chlorophényl)méthyl]azétidin-3-yl}-N-pyrid-3-yl-méthylsulfonamide.

6. Association selon les revendications 1 et 3 **caractérisée en ce que** le produit qui active la neurotransmission dopaminergique dans le cerveau est la lévodopa et le dérivé d'azétidine est le N-{1-[bis-(4-chlorophényl)méthyl]azétidin-3-yl}-N-(3,5-difluorophényl)-méthylsulfonamide.

7. Association selon l'une quelconque des revendications 1 à 6 pour son application à titre de médicament.

8. Association selon l'une quelconque des revendications 1 à 6 pour son application à titre de médicament dans le traitement de la maladie de Parkinson.

9. Association selon l'une quelconque des revendications 1 à 8 pour son usage simultané, séparé ou étalé dans le temps.

10. Composition pharmaceutique contenant un ou plusieurs produits activant la neurotransmission dopaminergique dans le cerveau et un ou plusieurs dérivés d'azétidine choisi parmi les composés suivants :
N-{1-[bis-(4-chlorophényl)méthyl]azétidin-3-yl}-N-pyrid-3-yl-méthylsulfonamide,
N-{1-[bis-(4-chlorophényl)méthyl]azétidin-3-yl}-N-(3,5-difluorophényl)-méthylsulfonamide,
avec un véhicule compatible et phamaceutiquement acceptable.

11. Composition pharmaceutique contenant un ou plusieurs produits activant la neurotransmission dopaminergique dans le cerveau et un dérivé d'azétidine :
N-{1-[bis-(4-chlorophényl)méthyl]azétidin-3-yl}-N-pyrid-3-yl-méthylsulfenamide,
avec un véhicule compatible et phamaceutiquement acceptable.

12. Composition pharmaceutique contenant un ou plusieurs produits activant la neurotransmission dopaminergique dans le cerveau et un dérivé d'azétidine:
N-{1-[bis-(4-chlorophényl)méthyl]azétidin-3-yl}-N-(3,5-difluorophényl)-méthylsulfonamide,
avec un véhicule compatible et phamaceutiquement acceptable.

13. Composition pharmaceutique selon l'une des revendications 10 à 12 **caractérisé en ce que** le produit qui active la neurotransmission dopaminergique dans le cerveau est choisi parmi les composés suivants :
bromocriptine, cabergoline, talipexole, Adrogolide, BAM-1110, duodopa, lévodopa, dopadose, CHF1301, CHF1512, PNU-95666, ropinirole, pramipexole , rotigotine, spheramine, TV1203, uridine, rasagiline, selegiline, SL340026, tolcapone, entacapone

14. Composition pharmaceutique selon la revendication 11 **caractérisée en ce que** le produit qui active la neurotransmission dopaminergique dans le cerveau est la lévodopa et le dérivé d'azétidine est le N-{1-[bis(4-chlorophényl)méthyl]azétidin-3-yl}-N-pyrid-3-yl-méthylsulfonamide.

15. Composition pharmaceutique selon la revendication 12 **caractérisée en ce que** le produit qui active la neurotransmission dopaminergique dans le cerveau est la lévodopa et le dérivé d'azétidine est le N-{1-[bis-(4-chlorophényl)méthyl]azétidin-3-yl}-N-(3,5-difluorophényl)-méthylsulfonamide.

16. Composition pharmaceutique selon l'une quelconque des revendications 10 à 15 comprenant de 0,1 à 500 mg de dérivé d'azétidine
N-{1-[bis-(4-chlorophényl)méthyl]azétidin-3-yl}-N-pyrid-3-yl-méthylsulfonamide.

17. Composition pharmaceutique selon l'une quelconque des revendications 10 à 15 comprenant de 0,1 à 500 mg de dérivé d'azétidine
N-{1-[bis-(4-chlorophényl)]méthyl]azétidin-3-yl}-N-(3,5-difluorophényl)-méthylsulfonamide.

## Claims

1. Combination of one or more products which activate dopaminergic neurotransmission in the brain and of one or more azetidine derivatives chosen from the following compounds:
N-{1-[bis(4-chlorophenyl)methyl]azetidin-3-yl}-N-pyrid-3-ylmethylsulphonamide,
N-{1-[bis(4-chlorophenyl)methyl]azetidin-3-yl}-N-(3,5-difluorophenyl)methylsulphonamide,
their pharmaceutically acceptable salts.

2. Combination of one or more products which activate dopaminergic neurotransmission in the brain and of one azetidine derivative:
N-{1-[bis(4-chlorophenyl)methyl]azetidin-3-yl}-N-pyrid-3-ylmethylsulphonamide,
their pharmaceutically acceptable salts.

3. Combination of one or more products which activate dopaminergic neurotransmission in the brain and of one azetidine derivative:
N-{1-[bis(4-chlorophenyl)methyl]azetidin-3-yl}-N-(3,5-difluorophenyl)methylsulphonamide,
their pharmaceutically acceptable salts.

4. Combination according to one of Claims 1 to 3, **characterized in that** the product which activates dopaminergic neurotransmission in the brain is chosen from the following compounds:
bromocriptine, cabergoline, Adrogolide, BAM-1110, duodopa, levodopa, dopadose, CHF1512, PNU-95666, ropinirole, pramipexole, rotigotine, spheramine, TV1203, uridine, rasagiline, selegiline, SL340026, tolcapone, entacapone.

5. Combination according to Claims 1 and 2, **characterized in that** the product which activates dopaminergic neurotransmission in the brain is levodopa and the CB1 antagonist is N-{1-[bis(4-chlorophenyl)-methyl]azetidin-3-yl}-N-pyrid-3-ylmethylsulphonamide.

6. Combination according to Claims 1 and 3, **characterized in that** the product which activates dopaminergic neurotransmission in the brain is levodopa and the azetidine derivative is N-{1-[bis(4-chlorophenyl)methyl]azetidin-3-yl}-N-(3,5-difluorophenyl)-methylsulphonamide.

7. Combination according to any one of Claims 1 to 6 for its application as a medicament.

8. Combination according to any one of Claims 1 to 6 for its application as a medicament in the treatment of Parkinson's disease.

9. Combination according to any one of Claims 1 to 8 for its use simultaneously, separately or spread out over time.

10. Pharmaceutical composition containing one or more products activating dopaminergic neurotransmission in the brain and one or more azetidine derivatives chosen from the following compounds:
N-{1-[bis(4-chlorophenyl)methyl]azetidin-3-yl}-N-pyrid-3-ylmethylsulphonamide,
N-{1-[bis(4-chlorophenyl)methyl]azetidin-3-yl}-N-(3,5-difluorophenyl)methylsulphonamide,
with a compatible and pharmaceutically acceptable vehicle.

11. Pharmaceutical composition containing one or more products activating dopaminergic neurotransmission in the brain and an azetidine derivative:
N-{1-[bis(4-chlorophenyl)methyl]azetidin-3-yl}-N-pyrid-3-ylmethylsulphonamide,
with a compatible and pharmaceutically acceptable vehicle.

12. Pharmaceutical composition containing one or more products activating dopaminergic neurotransmission in the brain and an azetidine derivative:
N-{1-[bis(4-chlorophenyl)methyl]azetidin-3-yl}-N-(3,5-difluorophenyl)methylsulphonamide,
with a compatible and pharmaceutically acceptable vehicle.

13. Pharmaceutical composition according to one of Claims 10 to 12, **characterized in that** the product which activates dopaminergic neurotransmission in the brain is chosen from the following compounds:
bromocriptine, cabergoline, talipexole, Adrogolide, BAM-1110, duodopa, levodopa, dopadose, CHF1301, CHF1512, PNU-95666, ropinirole, pramipexole, rotigotine, spheramine, TV1203, uridine, rasagiline, selegiline, SL340026, tolcapone, entacapone.

14. Pharmaceutical composition according to Claim 11, **characterized in that** the product which activates dopaminergic neurotransmission in the brain is levodopa and the azetidine derivative is N-{1-[bis(4-chlorophenyl)methyl]azetidin-3-yl}-N-pyrid-3-ylmethylsulphonamide.

15. Pharmaceutical composition according to Claim 12, **characterized in that** the product which activates dopaminergic neurotransmission in the brain is levodopa and the azetidine derivative is N-{1-[bis(4-chlorophenyl)methyl]azetidin-3-yl}-N-(3,5-difluorophenyl)-methylsulphonamide.

16. Pharmaceutical composition according to any one of Claims 10 to 15, comprising from 0.1 to 500 mg of azetidine derivative
N-{1-[bis(4-chlorophenyl)methyl]azetidin-3-yl}-N-pyrid-3-ylmethylsulphonamide.

17. Pharmaceutical composition according to any one of Claims 10 to 15, comprising from 0.1 to 500 mg of azetidine derivative
N-{1-[bis(4-chlorophenyl)methyl]azetidin-3-yl}-N-(3,5-difluorophenyl)methylsulphonamide.

## Patentansprüche

1. Assoziation von einem oder mehreren Produkten, welche die dopaminergische Neurotransmission im Gehirn aktivieren, und von einem oder mehreren Azetidin-Derivaten, ausgewählt unter den folgenden Verbindungen:
N-{1-[Bis-(4-Chlorphenyl)-methyl]-azetidin-3-yl}-N-pyrid-3-yl-methylsulfonamid,
N-{1-[Bis-(4-Chlorphenyl)-methyl]-azetidin-3-yl}-N-(3,5-difluorphenyl)-methylsulfonamid,
ihren pharmazeutisch akzeptablen Salzen.

2. Assoziation von einem oder mehreren Produkten, welche die dopaminergische Neurotransmission im Gehirn aktivieren, und von einem Azetidin-Derivat:
N-{1-[Bis-(4-Chlorphenyl)-methyl]-azetidin-3-yl}-N-pyrid-3-yl-methylsulfonamid,
ihren pharmazeutisch akzeptablen Salzen.

3. Assoziation von einem oder mehreren Produkten, welche die dopaminergische Neurotransmission im Gehirn aktivieren, und von einem Azetidin-Derivat:
N-{1-[Bis-(4-Chlorphenyl)-methyl]-azetidin-3-yl}-N-(3,5-difluorphenyl)-methylsulfonamid,
ihren pharmazeutisch akzeptablen Salzen.

4. Assoziation nach einem der Ansprüche 1 bis 3, **dadurch gekennzeichnet, daß** das Produkt, welches die dopaminergische Neurotransmission im Gehirn aktiviert, unter den folgenden Verbindungen ausgewählt wird:
Bromocriptin, Cabergolin, Adrogolid, BAM-1110, Duodopa, Levodopa, Dopados, CHF1512, PNU-95666, Ropinirol, Pramipexol, Rotigotin, Spheramin, TV1203, Uridin, Rasagilin, Selegilin, SL340026, Tolcapon, Entacapon.

5. Assoziation nach Anspruch 1 und 2, **dadurch gekennzeichnet, daß** das Produkt, welches die dopaminergische Neurotransmission im Gehirn aktiviert, das Levodopa ist, und der Antagonist CB1 das N-{1-[Bis-(4-Chlorphenyl)-methyl]-azetidin-3-yl}-N-pyrid-3-yl-methylsulfonamid ist.

6. Assoziation nach Anspruch 1 und 3, **dadurch gekennzeichnet, daß** das Produkt, welches die dopaminergische Neurotransmission im Gehirn aktiviert, das Levodopa ist, und das Azetidin-Derivat das N-{1-[Bis-(4-Chlorphenyl)-methyl]-azetidin-3-yl}-N-(3,5-difluorphenyl)-methylsulfonamid ist.

7. Assoziation nach irgendeinem der Ansprüche 1 bis 6 für ihre Anwendung als Arzneimittel.

8. Assoziation nach irgendeinem der Ansprüche 1 bis 6 für ihre Anwendung als Arzneimittel bei der Behandlung der Parkinson-Krankheit.

9. Assoziation nach irgendeinem der Ansprüche 1 bis 8 für ihre gleichzeitige, getrennte oder über die Zeit verteilte Anwendung.

10. Pharmazeutische Zusammensetzung, enthaltend ein oder mehrere Produkte, welche die dopaminergische Neurotransmission im Gehirn aktivieren, und ein oder mehrere Azetidin-Derivate, ausgewählt unter den folgenden Verbindungen:
N-{1-[Bis-(4-Chlorphenyl)-methyl]-azetidin-3-yl}-N-pyrid-3-yl-methylsulfonamid,
N-{1-[Bis-(4-Chlorphenyl)-methyl]-azetidin-3-yl}-N-(3,5-difluorphenyl)-methylsulfonamid,
mit einem kompatiblen und pharmazeutisch akzeptablen Vehikel.

11. Pharmazeutische Zusammensetzung, enthaltend ein oder mehrere Produkte, welche die dopaminergische Neurotransmission im Gehirn aktivieren, und ein Azetidin-Derivat:
N-{1-[Bis-(4-Chlorphenyl)-methyl]-azetidin-3-yl}-N-pyrid-3-yl-methylsulfonamid,
mit einem kompatiblen und pharmazeutisch akzeptablen Vehikel.

12. Pharmazeutische Zusammensetzung, enthaltend ein oder mehrere Produkte, welche die dopaminergische Neurotransmission im Gehirn aktivieren, und ein Azetidin-Derivat:
N-{1-[Bis-(4-Chlorphenyl)-methyl]-azetidin-3-yl}-N-(3,5-difluorphenyl)-methylsulfonamid,
mit einem kompatiblen und pharmazeutisch akzeptablen Vehikel.

13. Pharmazeutische Zusammensetzung nach einem der Ansprüche 10 bis 12, **dadurch gekennzeichnet, daß** das Produkt, welches die dopaminergische Neurotransmission im Gehirn aktiviert, unter den folgenden Verbindungen ausgewählt wird:
Bromocriptin, Cabergolin, Talipexol, Adrogolid, BAM-1110, Duodopa, Levodopa, Dopados, CHF1301, CHF1512, PNU-95666, Ropinirol, Pramipexol, Rotigotin, Spheramin, TV1203, Uridin, Rasagilin, Selegilin, SL340026, Tolcapon, Entacapon.

14. Pharmazeutische Zusammensetzung nach Anspruch 11, **dadurch gekennzeichnet, daß** das Produkt, welches die dopaminergische Neurotransmission im Gehirn aktiviert, das Levodopa ist, und das Azetidin-Derivat das N-{1-[Bis-(4-Chlorphenyl)-methyl]-azetidin-3-yl}-N-pyrid-3-yl-methylsulfonamid ist.

15. Pharmazeutische Zusammensetzung nach Anspruch 12, **dadurch gekennzeichnet, daß** das Produkt, welches die dopaminergische Neurotransmission im Gehirn aktiviert, das Levodopa ist, und das Azetidin-Derivat das N-{1-[Bis-(4-Chlorphenyl)-methyl]-azetidin-3-yl}-N-(3,5-difluorphenyl)-methylsulfonamid ist.

16. Pharmazeutische Zusammensetzung nach irgendeinem der Ansprüche 10 bis 15, umfassend 0,1 bis 500 mg Azetidin-Derivat N-{1-[Bis-(4-Chlorphenyl)-methyl]-azetidin-3-yl}-N-pyrid-3-yl-methylsulfonamid.

17. Pharmazeutische Zusammensetzung nach irgendeinem der Ansprüche 10 bis 15, umfassend 0,1 bis 500 mg Azetidin-Derivat N-{1-[Bis-(4-Chlorphenyl)-methyl]-azetidin-3-yl}-N-(3,5-difluorphenyl)-methylsulfonamid.
